# EUROPEAN PATENT APPLICATION

(11) **EP 2 229 964 A1**
(43) Date of publication of application: **22.09.2010**
(21) Application number: 10156173.6
(22) Date of filing: 11.03.2010
(51) Int. Cl.: A61L 29/16, A61K 9/14, A61K 31/155

(54) **Stable melt processable chlorhexidine compositions**

(30) Priority: 11.03.2009 US 401879
(71) Applicant: Teleflex Medical Incorporated, Durham, NC 27709 (US)
(72) Inventor: Do, Hiep, Sinking Spring, PA 19608 (US); Rosenblatt, Joel, Pottstown, PA 19465 (US); Okoh, Onajite, Reading, PA 19602 (US); Lu, Guangyu, Reading, PA 19605 (US); Sechrist, Kevin, Womelsdorf, PA 19567 (US)
(74) Representative: Stephen, Robert John

(57) **Abstract**

In a medical device having an antimicrobial agent, the medical device includes a base material and an amount of chlorhexidine or a pharmaceutically acceptable salt thereof disposed in the base material sufficient to reduce microbial growth. The base material is melt processed together with the chlorhexidine to generate the medical device which is substantially free of destabilized chlorhexidine.

## Description

### FIELD OF THE INVENTION

The present invention generally relates to medical devices having antimicrobial properties. More particularly, the present invention pertains to melt processable medical devices having antimicrobial properties and method of production thereof.

### BACKGROUND OF THE INVENTION

Medical devices are commonly used to facilitate care and treatment of patients undergoing surgical procedures. Examples of such devices include catheters, grafts, stents, sutures, and the like. Unfortunately, organisms such as bacteria and fungi may infiltrate and/or form biofilms on these medical devices which may be difficult to treat. Such contamination may lead to infections and cause discomfort or illness.

It is generally known that in various medical procedures, the use of medical devices having antimicrobial properties may reduce the incidence of infection in the patient. Typically, the antimicrobial agent is applied as a coating on the conventional medical device or the antimicrobial agent is infused into the conventional medical device by soaking the device in a solution of the antimicrobial agent. In these and other conventional methods of introducing the antimicrobial agent to the medical device, this extra step of coating or soaking takes time and increases costs.

In addition to the added step and increased production time, soaking and coating may not achieve relatively high concentrations of antibiotic in the base material of the medical device. For relatively short procedures having a duration of a few hours, this relatively low antibiotic concentration may be sufficient. However, for longer procedures lasting several days, the antibiotic present in conventional devices may be insufficient. As such, these conventional devices must be replaced frequently as the antibiotic falls below effective levels.

Accordingly, it is desirable to provide an antimicrobial medical device and/or method of introducing an antimicrobial agent to a medical device that is capable of overcoming the disadvantages described herein at least to some extent.

### SUMMARY OF THE INVENTION

The foregoing needs are met, to a great extent, by the present invention, wherein in one respect an antimicrobial medical device and method of introducing antimicrobial agent to the medical device is provided.

An embodiment of the present invention pertains to a medical device having an antimicrobial agent. The medical device includes a base material and an amount of chlorhexidine or a pharmaceutically acceptable salt thereof disposed in the base material sufficient to reduce microbial growth. The base material has a melt processable temperature below a temperature at which the chlorhexidine is destabilized.

Another embodiment of the present invention relates to a medical catheter including an elongated hollow tube, an exterior surface of the elongated hollow tube including a base material, and a chlorhexidine/fatty acid salt being disposed in the base material. The base material has a melt processable temperature below a temperature at which the chlorhexidine/fatty acid is destabilized.

Yet another embodiment of the present invention pertains to a medical catheter including an elongated hollow tube, an exterior surface of the elongated hollow tube including a polyvinylchloride base material, and a chlorhexidine/fatty acid salt being disposed in the polyvinylchloride base material in an amount sufficient to reduce microbial growth. The base material is melt processed at a temperature less than about 165°C together with the chlorhexidine/fatty acid salt to form the medical catheter which is substantially free of destabilized chlorhexidine.

Yet another embodiment of the present invention related to a medical catheter including an elongated hollow tube, an exterior surface of the elongated hollow tube including a polyurethane base material, and a chlorhexidine/fatty acid salt being disposed in the polyvinylchloride base material in an amount sufficient to reduce microbial growth. The polyurethane base material is melt processed at a temperature less than about 138°C together with the chlorhexidine/fatty acid salt to form the medical catheter which is substantially free of destabilized chlorhexidine.

Yet another embodiment of the present invention pertains to a method of fabricating a medical device having an antimicrobial agent. In this method, a base material is melted and the antimicrobial agent is added to the melted base material in an amount sufficient to reduce microbial growth. The antimicrobial agent includes chlorhexidine or a pharmaceutically acceptable salt thereof. The medical device is formed with the melted base material together with the chlorhexidine and is substantially free of destabilized chlorhexidine.

Yet another embodiment of the present invention related to a method of fabricating a medical device having an antimicrobial agent. In this method, a polyvinyl chloride base material is melted at less than about 165°C and the antimicrobial agent is added to the melted polyvinyl chloride base material in an amount sufficient to reduce microbial growth. The antimicrobial agent includes chlorhexidine or a pharmaceutically acceptable salt thereof The medical device is formed with the melted polyvinyl chloride base material together with the chlorhexidine and is substantially free of destabilized chlorhexidine.

Yet another embodiment of the present invention pertains to a method of fabricating a medical device having an antimicrobial agent. In this method, a polyurethane base material is melted at less than about 138°C and the antimicrobial agent is added to the melted polyvinyl chloride base material in an amount sufficient to reduce microbial growth. The antimicrobial agent includes chlorhexidine or a pharmaceutically acceptable salt thereof. The medical device is formed with the melted polyvinyl chloride base material together with the chlorhexidine and is substantially free of destabilized chlorhexidine.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is a high performance liquid chromatograph showing an analysis of a chlorhexidine diacetate standard in water/acetonitrile/methanol at a wavelength of 280 nanometers (nm).

FIG. 2 is a high performance liquid chromatograph showing an analysis of a chlorhexidine diacetate compounded in Tecothane 2095A at a melt temperature of 164°C at a wavelength of 280 nm.

FIG. 3 is a high performance liquid chromatograph showing an analysis of a chlorhexidine diacetate compounded in low melt temperature Tecoflex-93A at a melt temperature of 136°C at a wavelength of 280 nm.

FIG. 4 is a high performance liquid chromatograph showing an analysis of a chlorhexidine dodecanoate compounded in low melt temperature Tecoflex-93A at a melt temperature of 137°C at a wavelength of 280 nm.

FIG. 5 is a high performance liquid chromatograph showing an analysis of a chlorhexidine diacetate compounded in polyvinyl chloride (Shore A hardness of 65) at a melt temperature of 145°C at a wavelength of 280 nm.

FIG. 6 is a high performance liquid chromatograph showing an analysis of a chlorhexidine diacetate compounded in polyvinyl chloride (Shore A hardness of 85) at a melt temperature of 155°C at a wavelength of 280 nm.

FIG. 7 is a high performance liquid chromatograph showing an analysis of a chlorhexidine diacetate compounded in polyvinyl chloride (Shore A hardness of 85) at a melt temperature of 176°C at a wavelength of 280 nm.

### DETAILED DESCRIPTION

Embodiments of the invention provide infection resistant medical devices and methods of melt processing a base material with a chlorhexidine to generate the medical device. In various embodiments, the base material is selected and/or modified to include a melt processable temperature that is below a degradation temperature of the chlorhexidine. More particularly, a chlorhexidine and/or a pharmaceutically acceptable salt thereof may be uniformly incorporated into medical devices by directly melt processing it with polymers without degrading the chlorhexidine. In this regard, chlorhexidine degradation products may cause irritation or other such negative reactions in patients. By avoiding the production of these irritants, relatively high concentrations of chlorhexidine such as, for example up to about 30% (wt. chlorhexidine / wt. polymer), may be incorporated into a bulk material of the medical device. In addition, it is within the purview of this and other embodiments of the invention that other suitable agents may be incorporated into the bulk material. Examples of suitable agents includes other antibiotics, antiseptics, chemotherapeutics, antimicrobial peptides, mimetics, antithrombogenic, fibrinolytic, anticoagulants, anti-inflammatory, anti-pain, antinausea, vasodilators, antiproliferatives, antifibrotics, growth factors, cytokines, antibodies, peptide and peptide mimetics, nucleic acids, and/or the like.

Medical devices suitable for use with various embodiments of the invention may include catheters, tubes, sutures, non-wovens, meshes, drains, shunts, stents, foams etc. Other devices suitable for use with embodiments of the invention include those that would benefit from having a broad spectrum of antimicrobial and antifungal activity. Suitable methods of processing chlorhexidine and its salts in accordance with various embodiments of the invention may include compounding, extrusion, co-extrusion, injection molding, blow molding, compression molding, or other such 'hot melt' process. Benefits of one or more embodiments of this invention are the ability to form a device and at the same time incorporate high loadings of chlorhexidine without destabilizing or creating chlorhexidine degradation products. In this regard, as used herein, the term, 'destabilized' chlorhexidine refers to degraded, inactivated, or otherwise compromised chlorhexidine.

Forms of chlorhexidine suitable for use with embodiments of the invention include chlorhexidine base, pharmaceutically acceptable chlorhexidine salts such as, for example, diacetate, laurate (dodecanoate), palmitate (hexadecanoate), myristate (tetradecanoate), stearate (octadecanoate) and/or the like. Other examples of suitable chlorhexidine salts are to be found in U.S. Patent 6,706,024, entitled Triclosan-Containing Medical Devices, issued on March 16,2004, the disclosure of which is hereby incorporated in its entirety. In addition, while particular examples are made of chlorhexidine base, chlorhexidine diacetate, and chlorhexidine dodecanoate, embodiments of the invention are not limited to any one form. Instead, as used herein, the term, 'chlorhexidine' refers to any one or a mixture of chlorhexidine base, pharmaceutically acceptable chlorhexidine salts such as, for example, diacetate, dodecanoate, palmitate, myristate, stearate and/or the like. In general, suitable concentrations of chlorhexidine include a range from about 0.1% weight to weight (wt/wt) to about 30% wt/wt. More particularly, a suitable chlorhexidine range includes from about 3% wt/wt to about 20% wt/wt. Suitable base materials generally include pure and/or blended elastomers and/or polymer materials having melt processable temperatures of less than about 165 degrees Celsius (°C). More particularly, materials having a melt processable range of about 130°C to about 165° are suitable. Specific examples of suitable base materials include polyurethanes, polyvinylchlorides, thermoplastics such as, for example, fluoropolymers, vinyl polymers, polyolephins, copolymers, and/or the like. In other examples, polymers that are typically processed at temperatures relatively greater than 165°C may be modified to be melt processable at temperatures below 165°C. For example, the addition of plasticizing agents may suitably modify such polymers.

Polymer containing chlorhexidine maybe layered upon other bulk material to fabricate the medical device. For example, a material having a melt processable temperature greater than 165°C may be co-extruded with the polymer containing chlorhexidine.

As described herein, to validate some embodiments of the invention, we compounded several different polymers with various chlorhexidine salt combinations over a range of temperatures and allowed the blends to solidify. The chlorhexidine was then extracted using an organic solvent and analyzed for degradants by High Performance Liquid Chromatography (HPLC). Degradants were identified as new peaks in the chromatogram that were not present in a non-degraded control run under the same conditions. This method was used to identify upper processing temperature limits for stable melt processing of polymers such as polyurethanes and the like with chlorhexidine salts. We further performed our methods on a variety of commercially utilized polyurethanes and, surprisingly, observe that many of these commercial polyurethanes could not be melt processed below the upper processing limit. These unexpected results indicate that many commercially used polyurethanes were found to be unsuitable for stable melt processing with chlorhexidine.

In addition, our methods were utilized to define a processing temperature cutoff for vinyl polymers to enable stable melt processing with chlorhexidine. We found that many widely used vinyl polymers are not suitable for stable melt processing with chlorhexidine. Research performed according to embodiments of our invention further shows that, for the case of vinyl polymers, such as polyvinylchloride (PVC), the processing temperature can be lowered through the use ofplasticizing agents to enable stable processing with chlorhexidine. The addition of plasticizing agents is generally associated with a corresponding reduction in mechanical properties. However, we found that by laminating relatively soft PVC with chlorhexidine over a more rigid polymer, via co-extrusion or co-molding for example, the material characteristics such as excessive softness or lack of structural rigidity of PVC with chlorhexidine may be overcome. Furthermore, in some medical devices, antimicrobial protection may be most beneficial when present at the surfaces of the device. Therefore, this laminated construction may be advantageously employed in medical devices where the soft, chlorhexidine containing layers are disposed at the surface or exterior and mechanically stronger or more rigid layers are disposed below or to the interior of the medical device.

Moreover, the methods of our invention were utilized to define a processing temperature cutoff for a thermoplastic polyolephin elastomer (TPE). Again, our unexpected results indicate that many TPEs are not suitable for stable melt processing with chlorhexidine. Surprisingly, the upper processing temperature limits for stable melt processing are different for each class of polymer evaluated. It is also possible that specific salts of chlorhexidine may have different upper stable processing temperature limits. Accordingly, utilizing the methods and algorithms described herein, the upper stable melt processing temperature for other chlorhexidines in combination with other polymer chemistries could be defined.

In the following experiments, the use of specific polymers Tecothane®-2095A (Lubrizol, Cleveland, OH), Tecofiex®-93A (Lubrizol, Cleveland, OH) thermoplastic polyurethane (TPU), polytetramethyleneoxide (PTMO) (INVISTA, Wichita, KS), Versaflex® CL30 (GLS Inc., McHenry, Il), and Polyvinyl chloride having a flexural modulus or hardness of about Shore 65A and about Shore 85A (Colorite Polymers, Ridgefield, NJ) is specifically described. However, it is to be understood that any suitable polymer is within the scope of embodiments of this invention. Other suitable polymers include those manufactured by The Lubrizol Corp., Wickliffe, OH 44092, U.S.A., INVISTA S.à, r.l. Wichita, KS 67220, U.S.A., GLS Corp., McHenry, 11 60050, U.S.A., and Colorite Polymers, Ridgefield, NJ 07657, U.S.A. These polymers may be utilized in pure forms or combined with any suitable copolymer. Examples of suitable copolymers include one or more of silicone, fluoropolymers, polyurea-urethane, polyether-urethane, and the like. In addition, the chlorhexidine diacetate (George Uhe, Garfield, NJ), chlorhexidine dodecanoate (chlorhexidine laurate or chlorhexidine dilaurate) are specifically described. However, it is to be understood that any suitable chlorhexidine or salt thereof is within the scope of the embodiments of the invention. Other suitable chlorhexidine salts include chlorhexidine Myristate (chlorhexidine tetradecanoate), chlorhexidine palmitate (chlorhexidine hexadecanoate), chlorhexidine stearate (chlorhexidine octadecanoate), and various other chlorhexidines manufactured by the George Uhe Company Inc., Garfield, NJ 07026 U.S.A.

### METHODS

### EXAMPLE 1: Compound Tecothane®-2095A resin with 10% chlorhexidine diacetate

Tecothane®-2095A was coated with 5% w/w polytetramethyleneoxide (PTMO) of molecular weight (MW) = 1000 by mixing 45.1 gram (g) of PTMO with 900g Tecothane®-2095A. The PTMO coated resin and chlorhexidine diacetate were separately fed into an 18 millimeter (mm) Leistritz twin screw intermeshing extruder (Somerville, NJ) from K-Tron feeders (Pitman, NJ) at rates of 2.5 kilograms per hour (kg/hr) and 0.25 kg/hr, respectively. The extruder was set at 112 revolutions per minute (rpm) for screw speed and the barrel zone temperatures were set from 145°C thru 178°C. The extrudate was pelletized into small pellets.

### EXAMPLE 2: Compound low melt temperature Tecoflex-93A with 10% chlorhexidine diacetate

Low melting temperature Tecoflex-93A and chlorhexidine diacetate were separately fed into a18 mm Leistritz twin screw intermeshing extruder from K-tron feeders at rates of 1 kg/hr and 0.1 kg/hr, respectively. The barrel zone temperatures were set at 121°C for all zones. The extrudate was pelletized into small pellets.

### EXAMPLE 3: Synthesis of chlorhexidine dodecanoate

15.1g chlorhexidine base was slurried in 150 milliliters (ml) of isopropyl alcohol. 13.2g of dodecanoic acid was added to the slurry (2.1 molar equivalents). The solution went clear initially and later precipitation occurred. Precipitate was rinsed with 100ml isopropyl alcohol and filtered twice, after which it was vacuumed dried at 25°C for 24hrs. Yield was 88.7%.

### EXAMPLE 4: Compound low melt temperature Tecoflex-93A with 20% chlorhexidine dodecanoate

Low melting temperature Tecoflex-93A and chlorhexidine dodecanoate were separately fed into an 18 mm Leistritz twin screw intermeshing extruder from K-tron feeders at rates of 1 kg/hr and 0.2 kg/hr, respectively. The barrel zone temperatures were set at 121°C for all zones. The extrudate was pelletized into small pellets.

### EXAMPLE 5: Co-extruded chlorhexidine diacetate compounded resin into 7 French Gauge (Fr) single lumen tubing

A three layer construct (chlorhexidine layer-gentian violet (GV) layer-chlorhexidine layer) 7Fr single lumen tubing was co-extruded at temperature 121°C. Co-extruded tubing was also analyzed for chlorhexidine degradants.

### EXAMPLE 6: Compound Versaffex® CL30 with 10% chlorhexidine diacetate

Versaflex® CL30 and chlorhexidine diacetate were separately fed into an 18 mm Leistritz twin screw intermeshing extruder from K-tron feeders at rates of 2.5 kg/hr and 0.25 kg/hr, respectively. The barrel zone temperatures were set from 131°C thru 148°C. The extrudate was pelletized into small pellets.

### EXAMPLE 7: Compound PVC (65A and 85A) with 10% chlorhexidine diacetate

Separate samples of polyvinyl chloride (shore 65A and shore 85A respectively) and chlorhexidine diacetate were separately fed into an 18 mm Leistritz twin screw intermeshing extruder from K-tron feeders at rates of 2.0kg/hr and 0.2 kg/hr, respectively. The barrel zone temperatures were set from 140°C thru 155°C. The extrudate was pelletized into small pellets.

### EXAMPLE 8: Characterization of Chlorhexidine Extracted From Samples via HPLC

Chlorhexidine diacetate content of the prepared samples was extracted with 1:1 tetrahydrofuran (THF): H₂O and analyzed on the Agilent Eclipse XDB-CN 5u 4.6x150mm column with guard column. Briefly, 2 centimeter (cm) sample segments were extracted with 5mL of THF and 5mL of H₂O, vortexed, and centrifuged. HPLC analysis was run on an Agilent Eclipse XDB-CN 5u 4.6x150mm column and 4.6 x 12.5mm Eclipse XDB-CN guard column, with a mixture of deionized water, acetonitrile, and trifluoroacetic acid as the mobile phase. Concentrations of the analytes were determined via calibration curves.

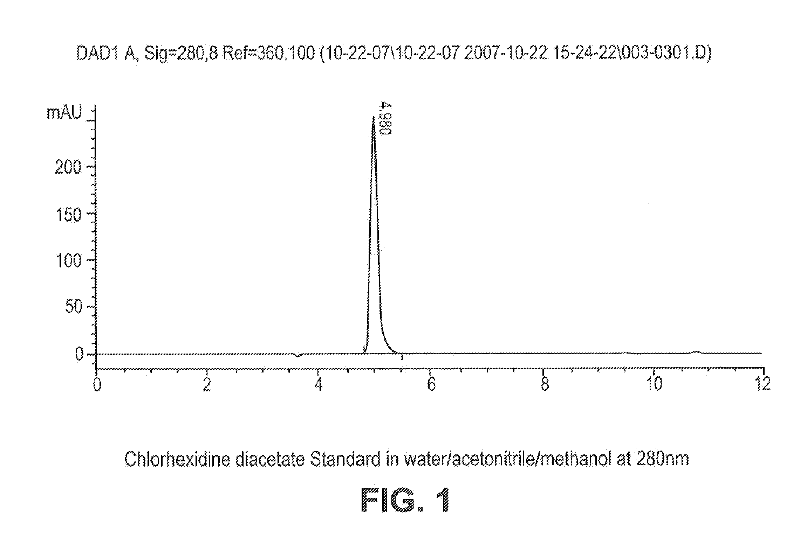

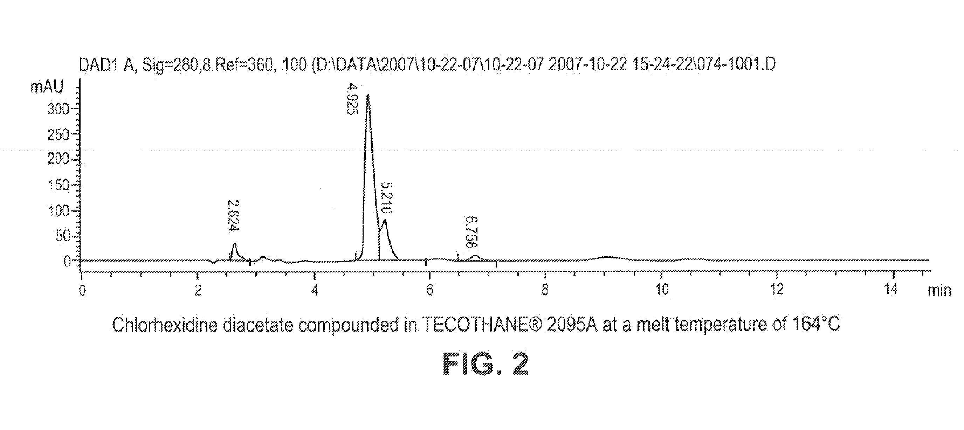

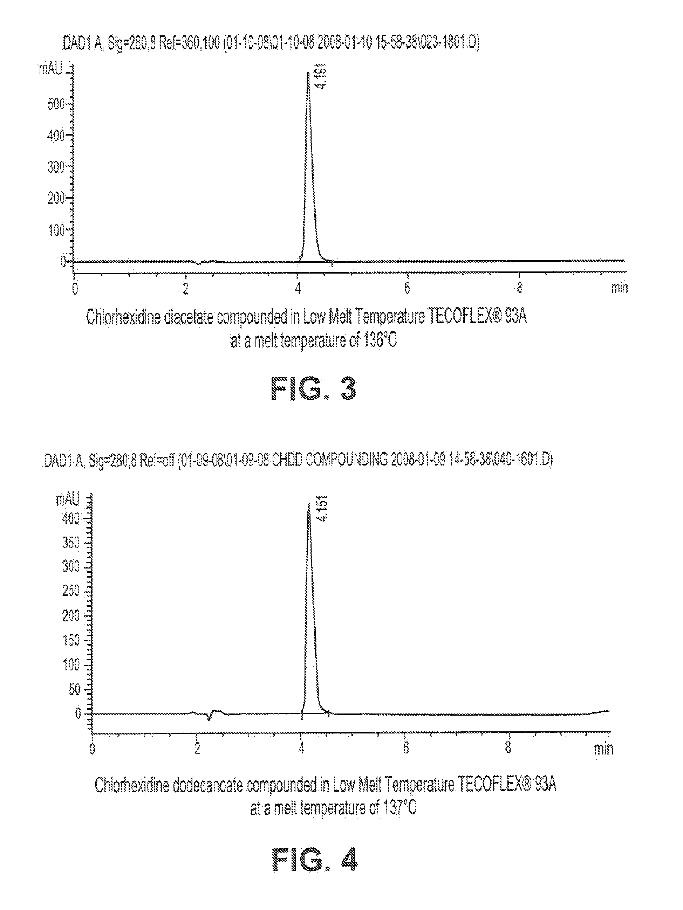

In the following Results section, a positive control showing high performance liquid chromatography analysis of non-degraded chlorhexidine is illustrated in FIG. 1. A negative control showing degradation products generated by exposing chlorhexidine to elevated temperatures is illustrated in FIG. 2. FIGS. 3 to 7 present high performance liquid chromatographs of samples prepared as described herein.

### RESULTS

FIG. 1 is a high performance liquid chromatograph showing an analysis of a chlorhexidine diacetate standard in water/acetonitrile/methanol. In the absence of exposure to elevated temperatures, chlorhexidine diacetate generates a single peak. As shown in FIG. 1, ultra violet (UV) absorbance of a chlorhexidine standard was measured at 280 nanometer (nm) wavelength and its elution time or relative retention time was determined to be approximately 4.980. The elution time is in minutes and the elution time of a chlorhexidine standard is used to calculate a relative retention time (RRT) of the degradants. In general, the RRT equals the elution time of the degradant divided by the elution time of the chlorhexidine standard.

FIG. 2 is a high performance liquid chromatograph showing an analysis of a chlorhexidine diacetate compounded in Tecothane 2095A at a melt temperature of 164°C at a wavelength of 280 nm. As a result of exposure to elevated temperatures, chlorhexidine diacetate degrades into several products represented by the peaks shown in FIG. 2. As shown in FIG. 2, chlorhexidine degradant RRT were determined to be approximately 0.6,1.3, and 1.6 at 280 nm wavelength.

In addition to the experimental conditions described with reference to FIG. 2, chlorhexidine diacetate compounded in Tecothane 2095A was subjected to melt temperatures ranging from about 139°C to about 172°C and the concentration of the analytes was determined via a calibration curve as described in the following Table 1.

**TABLE 1: Chlorhexidine diacetate (CHA) extracted from Tecothane®-2095A compounded resin**

| **Condition** | **Melt Temp (°C)** | **4.1 peak (CHA)** | **RRT 0.6 (degradant)** | **RRT 1.3 (degradant)** | **RRT 1.6 (degradant)** |
|---|---|---|---|---|---|
| 1 | 139 | 89 | 3 | 8 | 1 |
| 2 | 142 | 88 | 3 | 8 | 1 |
| 3 | 144 | 84 | 4 | 11 | 1 |
| 4 | 172 | 77 | 7 | 13 | 3 |

As shown in Table 1, chlorhexidine extracted from the samples prepared as described in Example 1 was characterized by HPLC and summarized as percentages of each peak recovered. Three additional chlorhexidine degradants were detected at RRTs of 0.6, 1.3, and 1.6.

FIG. 3 is a high performance liquid chromatograph showing an analysis of a chlorhexidine diacetate compounded in low melt temperature Tecoflex-93A at a melt temperature of 136°C at a wavelength of 280 nm. As shown in FIG. 3, chlorhexidine diacetate compounded in low melt temperature Tecoflex-93A did not degrade as a result of being processed at 136°C.

In addition to the experimental conditions described with reference to FIG. 3, chlorhexidine diacetate compounded in low melt temperature Tecoflex-93A was subjected to melt temperatures ranging from about 131°C to about 137°C. The concentration of the analytes was determined via a calibration curve as described in the following Table 2.

**TABLE 2: Chlorhexidine diacetate (CHA) extracted from low melt temperature (LMT) Tecoflex-93A compounded resin**

| **Condition** | **Melt Temp (°C)** | **4.289 peak (CHA)** | **RRT 0.6 (degradant)** | **RRT 1.3 (degradant)** | **RRT 1.6 (degradant)** |
|---|---|---|---|---|---|
| 1 | 131 | 100 | Not detected | Not detected | Not detected |
| 2 | 137 | 100 | Non-detected | Not detected | Not detected |

As shown in Table 2, chlorhexidine extracted from the samples prepared as described in example 2 does not exhibit additional chlorhexidine degradation peaks for chlorhexidine diacetate in these samples. Accordingly, the stable processing temperature limit for chlorhexidine diacetate with polyurethanes appears to be about 137°C.

FIG. 4 is a high performance liquid chromatograph showing an analysis of a chlorhexidine dodecanoate compounded in low melt temperature Tecoflex-93A at a melt temperature of 137°C at a wavelength of 280 nm. As shown in FIG. 4, chlorhexidine dodecanoate compounded in low melt temperature Tecoflex-93A did not degrade as a result of being processed at 137°C.

In addition to the experimental conditions described with reference to FIG. 4, chlorhexidine dodecanoate compounded in low melt temperature Tecoflex-93A was subjected to melt temperatures ranging from about 132°C to about 136°C. The concentration of the analytes was determined via a calibration curve as described in the following Table 3.

**TABLE 3: Chlorhexidine dodecanoate (CHDD) extracted from LMT Tecoflex-93A compounded resin**

| **Condition** | **Melt Temp (°C)** | **4.289 peak (CHDD)** | **RRT 0.6 (degradant)** | **RRT 1.3 (degradant)** | **RRT 1.6 (degradant)** |
|---|---|---|---|---|---|
| 1 | 132 | 100 | Non-detected | Not detected | Not detected |
| 2 | 136 | 100 | Non-detected | Not detected | Not detected |

As shown in Table 3, chlorhexidine extracted from the samples prepared as described in Example 3 does not exhibit additional chlorhexidine degradation peaks for chlorhexidine dodecanoate detected in these samples. Thus, the stable processing temperature limit for chlorhexidine dodecanoate with polyurethanes appears to be at least 136°C to about 137°C.

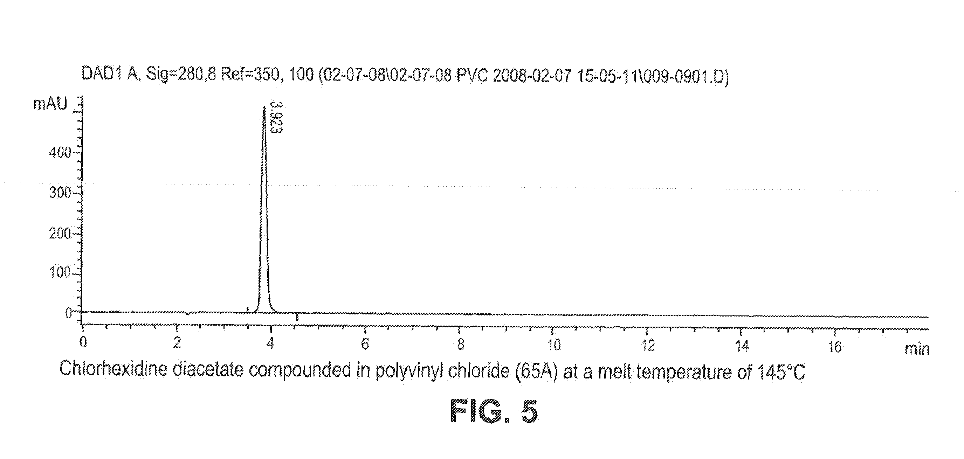

FIG. 5 is a high performance liquid chromatograph showing an analysis of a chlorhexidine diacetate compounded in polyvinyl chloride (Shore A hardness of 65) at a melt temperature of 145°C at a wavelength of 280 nm. As shown in FIG. 5, chlorhexidine diacetate compounded in polyvinyl chloride (Shore A hardness of 65) did not degrade as a result of being processed at 145°C.

In addition to the experimental conditions described with reference to FIG. 5, chlorhexidine diacetate compounded in polyvinyl chloride (Shore A hardness of 65) was subjected to a melt temperature of about 135°C. The concentration of the analytes was determined via a calibration curve as described in the following Table 4.

**TABLE 4: CHA extracted from co-extruded 7Fr single lumen**

| **Condition** | **Melt Temp (°C)** | **4.289 peak (CHA)** | **RRT 0.6 (degradant)** | **RRT 1.3 (degradant)** | **RRT 1.6 (degradant)** |
|---|---|---|---|---|---|
| 1 | 135 | 100 | Not detected | Not detected | Not detected |

As shown in Table 4, chlorhexidine extracted from the samples prepared as described in Example 4 does not exhibit additional chlorhexidine degradation peaks for chlorhexidine diacetate in this sample. Thus, the stable processing temperature limit for chlorhexidine diacetate with polyurethanes appears to be at least about 135°C.

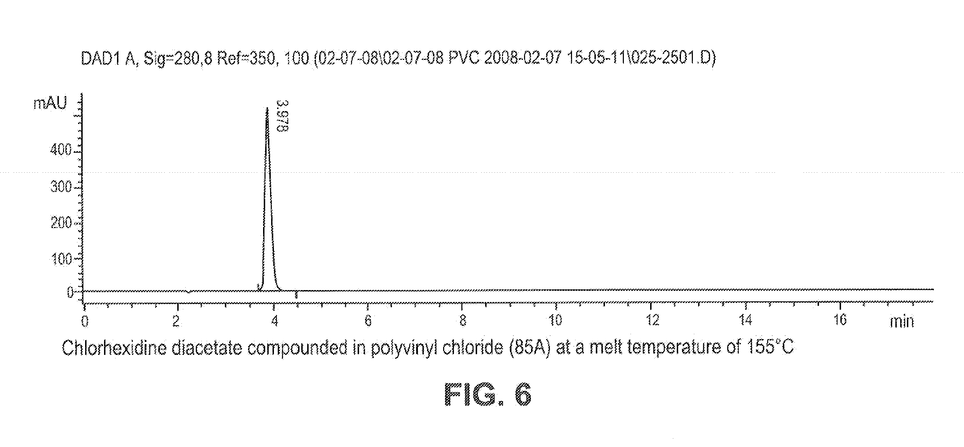

FIG. 6 is a high performance liquid chromatograph showing an analysis of a chlorhexidine diacetate compounded in polyvinyl chloride (Shore A hardness of 85) at a melt temperature of 155°C at a wavelength of 280 nm. As shown in FIG. 6, chlorhexidine diacetate compounded in polyvinyl chloride (Shore A hardness of 85) did not degrade as a result of being processed at 155°C.

In addition to the experimental conditions described with reference to FIG. 6, chlorhexidine diacetate compounded in polyvinyl chloride (Shore A hardness of 85) was subjected to melt temperatures ranging from about 101 °C to about 161°C. The concentration of the analytes was determined via a calibration curve as described in the following Table 5.

**TABLE 5: CHA extracted from Versaflex CL30 compounded resin**

| Condition | Melt Temp (°C) | 4.1 peak (CHA) | RRT 0.6 (degradant) | RRT 1.3 (degradant) | RRT 1.6 (degradant) |
|---|---|---|---|---|---|
| 1 | 101 | 100 | Not detected | Not detected | Not detected |
| 2 | 121 | 100 | Not detected | Not detected | Not detected |
| 3 | 129 | 100 | Not detected | Not detected | Not detected |
| 4 | 139 | 100 | Not detected | Not detected | Not detected |
| 5 | 144 | 100 | Not detected | Not detected | Not detected |
| 6 | 149 | 100 | Not detected | Not detected | Not detected |
| 7 | 153 | 100 | Not detected | Not detected | Not detected |
| 8 | 161 | 100 | Not detected | Not detected | Not detected |

As shown in Table 5, chlorhexidine extracted from the samples prepared as described in Example 5 did not degrade at a processing temperature up to 161°C. Versaflex CL30 is a mixture or alloy of polyolefins. These results show chlorhexidine is thermally stable at processing temperatures up to about 161°C with polyolefin thermoplastic elastomers. These findings are unexpected and surprising in light of the relatively lower maximum processing temperature for polyurethanes. These finding indicate that the thermal stability of chlorhexidine is temperature and materials dependent.

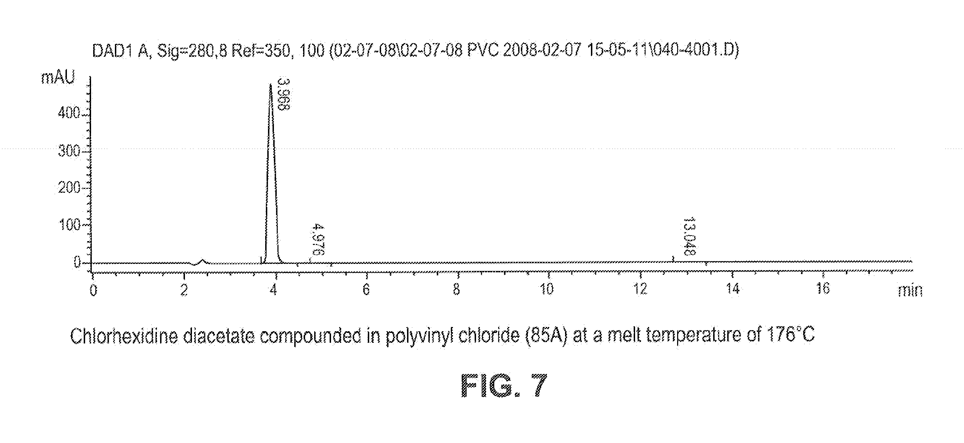

FIG. 7 is a high performance liquid chromatograph showing an analysis of a chlorhexidine diacetate compounded in polyvinyl chloride (Shore A hardness of 85) at a melt temperature of 176°C at a wavelength of 280 nm. As shown in FIG. 7, chlorhexidine diacetate compounded in polyvinyl chloride (Shore A hardness of 85) and processed at 176°C generated a trace or threshold amount (e.g., less than 1%).

In addition to the experimental conditions described with reference to FIG. 7, chlorhexidine diacetate compounded in polyvinyl chloride (Shore A hardness of 60 & 85) was subjected to melt temperatures ranging from about 145°C to about 176°C. The concentration of the analytes was determined via a calibration curve as described in the following Table 6.

**TABLE 6: CHA extracted from PVC (60A & 85A) compounded resin**

| Condition | Melt temp (°C) | % Peak area CHA | % Peak area (degradant) |
|---|---|---|---|
| PVC 60A | | | |
| 1 | 145 | 100 | Not detected |
| 2 | 154 | 100 | Not detected |
| 3 | 159 | 100 | Not detected |
| 4 | 165 | 100 | Not detected |
| *5* | *171* | *99. 6* | *0.4* |
| PVC 85A | | | |
| 1 | 155 | 100 | Not detected |
| 2 | 159 | 100 | Not detected |
| 3 | 163 | 100 | Not detected |
| *4* | *167* | *99.6* | *0.4* |
| *5* | *170* | 99.7 | *0.3* |
| *6* | *176* | *99.3* | *0.*7 |

As shown in Table 6, chlorhexidine extracted from the samples prepared as described in example 6 did not degrade at processing temperatures up to approximately 165°C. PVC (60A & 85A) materials are mixtures or alloys of different polyvinyl chloride compositions. These results show chlorhexidine is thermally stable at processing temperatures up to approximately 165°C with vinyl polymers. These findings are unexpected and surprising in light of the relatively lower maximum processing temperature for polyurethanes. These findings present further evidence that the thermal stability of chlorhexidine is temperature and materials dependent.

A significant benefit of various embodiments of the invention is the ability to fabricate a chlorhexidine laden polymer structure in a single step. That is, the subsequent processing to introduce antibiotic agents into the extruded or molded structure that is performed during the fabrication of conventional medical devices may be omitted. In so doing, time and money may be saved.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

Embodiments of the invention can be summarized in the following statements:
A. A medical device having an antimicrobial agent, the medical device comprising:
   a base material; and
   an amount of chlorhexidine or a pharmaceutically acceptable salt thereof disposed in the base material sufficient to reduce microbial growth, the base material having a melt processable temperature below a temperature at which chlorhexidine is destabilized.
B. The medical device according to Statement A, wherein the base material is melt processed together with the chlorhexidine to generate the medical device which is substantially free of destabilized chlorhexidine.
C. The medical device according to Statement A, further comprising:
   a first layer including a core material; and
   a second layer including the base material.
D. The medical device according to Statement C, further comprising:
   a plurality of layers of the core material; and
   a plurality of layers of the base material.
E. The medical device according to Statement A, further comprising:
   a first region including a core material; and
   a second region including the base material.
F. The medical device according to Statement E, further comprising:
   a plurality of regions of the core material; and
   a plurality of regions of the base material.
G. The medical device according to Statement A, wherein the base material further comprises:
   a copolymer including one or more of silicone, fluoropolymers, polyurea-urethane, and polyether-urethane.
H. The medical device according to Statement A, wherein the base material comprises:
   a polyurethane.
      1. The medical device according to Statement H, wherein the polyurethane is melt processable at or below a temperature of about 137°C.
J. The medical device according to Statement H, wherein the polyurethane is a polyurethane foam.
K. The medical device according to Statement A, wherein the base material comprises:
   a polyvinylchloride.
L. The medical device according to Statement K, wherein the polyvinylchloride is melt processable at or below a temperature of about 165°C.
M. The medical device according to Statement A, wherein the base material comprises:
   a blend of polymers.
N. The medical device according to Statement B, wherein the base material is melt processed from about 130°C to about 165°C.
O. The medical device according to Statement A, wherein the base material comprises:
   a thermoplastic.
P. The medical device according to Statement A, wherein the antimicrobial agent comprises:
   a chlorhexidine diacetate.
Q. The medical device according to Statement A, wherein the antimicrobial agent comprises:
   a chlorhexidine/fatty acid salt, wherein the chlorhexidine/fatty acid salt is a neutralization product of chlorhexidine base and a fatty acid having between 12 and 18 carbon atoms.
R. The medical device according to Statement Q, wherein the chlorhexidine/fatty acid salt further comprises a straight chain fatty acid.
S. The medical device according to Statement R, wherein the fatty acid comprises between 12 and 16 carbon atoms.
T. The medical device according to Statement S, wherein the chlorhexidine/fatty acid salt comprises a chlorhexidine Laurate (chlorhexidine dodecanoate).
U. The medical device according to Statement S, wherein the chlorhexidine/fatty acid salt comprises a chlorhexidine Palmitate (chlorhexidine hexadecanoate).
V. The medical device according to Statement A, further comprising:
   a mixture of chlorhexidine base and a pharmaceutically acceptable salt thereof.
W. The medical device according to Statement A, further comprising:
   a mixture of pharmaceutically acceptable chlorhexidine salts.
X. The medical device according to Statement A, further comprising:
   a bioactive agent including one or more of an antibiotic, antiseptic, chemotherapeutic, antimicrobial peptide, mimetic, antithrombogenic, fibrinolytic, anticoagulant, anti-inflammatory, anti-pain, antinausea, vasodilator, antiproliferative, antifibrotic, growth factor, cytokine, antibody, peptide and peptide mimetics, and nucleic acid.
Y. A medical catheter comprising:
   an elongated hollow tube;
   an exterior surface of the elongated hollow tube including a base material; and
   a chlorhexidine/fatty acid salt being disposed in the base material, the base material having a melt processable temperature below a temperature at which chlorhexidine/fatty acid salt is destabilized.
Z. The medical device according to Statement Y, wherein the base material is melt processed together with the chlorhexidine/fatty acid salt to form the medical catheter which is substantially free of destabilized chlorhexidine.
AA. The medical catheter according to Statement Y, further comprising:
   a first layer including a core material; and
   a second layer including the base material.
AB. The medical catheter according to Statement Y, further comprising:
   a plurality of layers of the core material; and
   a plurality of layers of the base material.
AC. The medical catheter according to Statement Y, further comprising:
   a first region including a core material; and
   a second region including the base material.
AD. The medical catheter according to Statement AC, further comprising:
   a plurality of regions of the core material; and
   a plurality of regions of the base material.
AE. The medical catheter according to Statement Y, wherein the base material further comprises:
   a copolymer including one or more of silicone, fluoropolymers, polyurea-urethane, and polyether-urethane.
AF. The medical catheter according to Statement Y, wherein the base material comprises:
   a polyurethane.
AG. The medical catheter according to Statement AF, wherein the polyurethane is a polyurethane foam.
AH. The medical catheter according to Statement Y, wherein the base material comprises:
   a thermoplastic.
AI. The medical catheter according to Statement Y, wherein the antimicrobial agent comprises:
   a chlorhexidine diacetate.
AJ. The medical catheter according to Statement Y, wherein the antimicrobial agent comprises:
   a chlorhexidine/fatty acid salt, wherein the chlorhexidine/fatty acid salt is a neutralization product of chlorhexidine base and a fatty acid having between 12 and 18 carbon atoms.
AK. The medical catheter according to Statement AJ, wherein the chlorhexidine/fatty acid salt further comprises a straight chain fatty acid.
AL. The medical catheter according to Statement AK, wherein the fatty acid comprises between 12 and 16 carbon atoms.
AM. The medical catheter according to Statement AL, wherein the chlorhexidine/fatty acid salt comprises a chlorhexidine Laurate (chlorhexidine dodecanoate).
AN. The medical catheter according to Statement Y, further comprising:
   a bioactive agent including one or more of an antibiotic, antiseptic, chemotherapeutic, antimicrobial peptide, mimetic, antithrombogenic, fibrinolytic, anticoagulant, anti-inflammatory, anti-pain, antinausea, vasodilator, antiproliferative, antifibrotic, growth factor, cytokine, antibody, peptide and peptide mimetics, and nucleic acid.
AO. A medical device comprising:
   a polyvinylchloride base material; and
   a chlorhexidine base and/or chlorhexidine/fatty acid salt being disposed in the polyvinylchloride base material in an amount sufficient to reduce microbial growth, wherein the base material is melt processed at a temperature less than about 165°C together with the chlorhexidine base and/or chlorhexidine/fatty acid salt to form the medical device which is substantially free of destabilized chlorhexidine.
AP. A medical device comprising:
   a polyurethane base material; and
   a chlorhexidine base and/or chlorhexidine/fatty acid salt being disposed in the polyurethane base material in an amount sufficient to reduce microbial growth, wherein the polyurethane base material is melt processed at a temperature less than about 138°C together with the chlorhexidine base and/or chlorhexidine/fatty acid salt to form the medical device which is substantially free of destabilized chlorhexidine.
AQ. A medical catheter comprising:
   an elongated hollow tube;
   an exterior surface of the elongated hollow tube including a polyvinylchloride base material; and
   a chlorhexidine/fatty acid salt being disposed in the polyvinylchloride base material in an amount sufficient to reduce microbial growth, wherein the base material is melt processed at a temperature less than about 165°C together with the chlorhexidine/fatty acid salt to form the medical catheter which is substantially free of destabilized chlorhexidine.
AR. The medical catheter according to Statement AQ, further comprising:
   a first layer including a core material; and
   a second layer including the polyvinylchloride base material.
AS. A medical catheter comprising:
   an elongated hollow tube;
   an exterior surface of the elongated hollow tube including a polyurethane base material; and
   a chlorhexidine/fatty acid salt being disposed in the polyurethane base material in an amount sufficient to reduce microbial growth, wherein the polyurethane base material is melt processed at a temperature less than about 138°C together with the chlorhexidine/fatty acid salt to form the medical catheter which is substantially free of destabilized chlorhexidine.
AT. A method of fabricating a medical device having an antimicrobial agent, the method comprising:
   melting a base material;
   adding the antimicrobial agent to the melted base material in an amount sufficient reduce microbial growth, wherein the antimicrobial agent includes chlorhexidine or a pharmaceutically acceptable salt thereof; and
   forming the medical device with the melted base material/chlorhexidine, wherein the medical device is substantially free of destabilized chlorhexidine.
AU. The method according to Statement AT, further comprising:
   heating the base material to a temperature below about 167°C.
AV. The method according to Statement AU, further comprising:
   heating the base material to a temperature range between about 130°C to about 165°C.
AW. The method according to Statement AT, further comprising:
   selecting the base material.
AX. The method according to Statement AW, further comprising:
   selecting a polyurethane as the base material.
AY. The method according to Statement AW, further comprising:
   selecting a thermoplastic as the base material.
AZ. The method according to Statement AW, further comprising:
   determining a maximum process temperature at which destabilized chlorhexidine is generated; and
   melting the base material at a temperature below the maximum process temperature.
BA. The method according to Statement AT, further comprising:
   selecting a chlorhexidine diacetate for the antimicrobial agent.
BB. The method according to Statement AT, further comprising:
   selecting a chlorhexidine/fatty acid salt for the antimicrobial agent, wherein the chlorhexidine/fatty acid salt is a neutralization product of chlorhexidine base and a fatty acid having between 12 and 18 carbon atoms.
BC. The method according to Statement BB, further comprising:
   selecting the chlorhexidine/fatty acid salt for the antimicrobial agent, wherein the fatty acid comprises a straight chain fatty acid.
BD. The method according to Statement BB, further comprising:
   selecting the chlorhexidine/fatty acid salt for the antimicrobial agent, wherein the fatty acid comprises between 12 and 16 carbon atoms.
BE. The method according to Statement BB, further comprising:
   selecting the chlorhexidine/fatty acid salt for the antimicrobial agent, wherein the chlorhexidine/fatty acid salt comprises a chlorhexidine Laurate (chlorhexidine dodecanoate).
BF. The method according to Statement AT, further comprising:
   forming a first layer of the medical device from a core material; and
   forming a second layer of the medical device with the melted base material/chlorhexidine.
BG. The method according to Statement BF, further comprising:
   co-extruding the first layer and second layer.
BH. A method of fabricating a medical device having an antimicrobial agent, the method comprising:
   melting a polyvinyl chloride base material at less than about 165°C;
   adding the antimicrobial agent to the melted polyvinyl chloride base material in an amount sufficient reduce microbial growth, wherein the antimicrobial agent includes chlorhexidine or a pharmaceutically acceptable salt thereof; and
   forming the medical device with the melted polyvinyl chloride base material/chlorhexidine, wherein the medical device is substantially free of destabilized chlorhexidine.
BI. A method of fabricating a medical device having an antimicrobial agent, the method comprising:
   melting a polyurethane base material at less than about 138°C;
   adding the antimicrobial agent to the melted polyurethane base material in an amount sufficient reduce microbial growth, wherein the antimicrobial agent includes chlorhexidine or a pharmaceutically acceptable salt thereof; and
   forming the medical device with the melted polyurethane base material/chlorhexidine, wherein the medical device is substantially free of destabilized chlorhexidine.

## Claims

1. A medical device comprising:
a base material; and
an amount of chlorhexidine or pharmaceutically acceptable salt thereof disposed in the base material sufficient to reduce microbial growth, the base material having a melt processable temperature below a temperature at which chlorhexidine is destabilized.

2. The medical device according to claim 1,wherein the base material is melt processed together with the chlorhexidine to generate the medical device which is substantially free of destabilized chlorhexidine.

3. The medical device according to claim 1, further comprising:
a first layer or region including a core material; and
a second layer or region including the base material; optionally
further comprising:
a plurality of layers or regions of the core material; and
a plurality of layers or regions of the base material.

4. The medical device according to claim 1, wherein the base material further comprises:
a copolymer including one or more of silicone, fluoropolymers, polyurea-urethane, and polyether-urethane.

5. The medical device according to claim 1, wherein the base material comprises:
a thermoplastic, a polyurethane, a polyurethane foam, a polyvinylchloride, or a blend of polymers.

6. The medical device according to claim 5, wherein the base material is a polyurethane and is melt processable at or below a temperature of about 137°C.

7. The medical device according to claim 5, wherein the base material is a polyvinylchloride and is melt processable at or below a temperature of about 165°C.

8. The medical device according to claim 1, wherein the base material is melt processed from about 130°C to about 165°C.

9. The medical device according to claim 1, wherein the chlorhexidine or pharmaceutically acceptable salt thereof is selected from the group consisting of:
a chlorhexidine diacetate;
a chlorhexidine/fatty acid salt, wherein the chlorhexidine/fatty acid salt is a neutralization product of chlorhexidine base and a fatty acid having between 12 and 18 carbon atoms;
a chlorhexidine/fatty acid salt, wherein the chlorhexidine/fatty acid salt further comprises a straight chain fatty acid;
a chlorhexidine/fatty acid salt, wherein the fatty acid comprises between 12 and 16 carbon atoms;
a chlorhexidine/fatty acid salt, wherein the chlorhexidine/fatty acid salt comprises a chlorhexidine Laurate (chlorhexidine dodecanoate);
a chlorhexidine/fatty acid salt, wherein the chlorhexidine/fatty acid salt comprises a chlorhexidine Palmitate (chlorhexidine hexadecanoate);
a mixture of chlorhexidine base and a pharmaceutically acceptable salt thereof; and
a mixture of pharmaceutically acceptable chlorhexidine salts.

10. The medical device according to claim 1, further comprising:
a bioactive agent including one or more of an antibiotic, antiseptic, chemotherapeutic, antimicrobial peptide, mimetic, antithrombogenic, fibrinolytic, anticoagulant, anti-inflammatory, anti-pain, antinausea, vasodilator, antiproliferative, antifibrotic, growth factor, cytokine, antibody, peptide and peptide mimetics, and nucleic acid.

11. A medical device, which is a catheter, according to any of claims 1-10 further comprising:
an elongated hollow tube; and
an exterior surface of the elongated hollow tube including the base material.

12. The medical device according to any of claims 1-11 being fabricated according to the method steps of:
melting the base material;
adding the chlorhexidine or a pharmaceutically acceptable salt thereof to the melted base material in an amount sufficient reduce microbial growth; and
forming the medical device with the melted base material/chlorhexidine, wherein the medical device is substantially free of destabilized chlorhexidine.

13. The medical device according to claim 12, being fabricated according to the method step of:
heating the base material to a temperature below about 167°C; optionally
heating the base material to a temperature range between about 130°C to about 165°C.

14. The medical device according to claim 12, being fabricated according to the method steps of:
forming a first layer of the medical device from a core material;
forming a second layer of the medical device with the melted base material/chlorhexidine; and
optionally co-extruding the first layer and second layer.

15. The medical device according to claim 12, being fabricated according to the method steps of:
determining a maximum process temperature at which destabilized chlorhexidine is generated; and
melting the base material at a temperature below the maximum process temperature.
